# EUROPÄISCHE PATENTSCHRIFT

(11) **EP 0 863 869 B1**
(45) Veröffentlichungstag und Bekanntmachung des Hinweises auf die Patenterteilung: **16.08.2000**
(21) Anmeldenummer: 96938155.7
(22) Anmeldetag: 08.11.1996
(51) Int. Cl.: C07C 303/40, C07C 311/29, C07C 309/87

(54) **VERFAHREN ZUR HERSTELLUNG VON 2-TRIFLUORMETHOXY-BENZOLSULFONAMID**
METHOD OF PREPARING 2-TRIFLUOROMETHOXYBENZENE SULPHONAMIDE
PROCEDE DE PRODUCTION DE 2-TRIFLUOROMETHOXY-BENZOLSULFONAMIDE

(30) Priorität: 21.11.1995 DE 19543323
(43) Veröffentlichungstag der Anmeldung: 16.09.1998
(73) Patentinhaber: BAYER AG, 51368 Leverkusen (DE)
(72) Erfinder: LANTZSCH, Reinhard, D-42115 Wuppertal (DE); MARHOLD, Albrecht, D-51373 Leverkusen (DE); KYSELA, Ernst, D-51427 Bergisch Gladbach (DE)
(86) Internationale Anmeldenummer: EP9604895
(87) Internationale Veröffentlichungsnummer: WO9719056

(56) Entgegenhaltungen:
- US-A- 2 809 194
- JOURNAL OF ORGANIC CHEMISTRY OF THE USSR, Bd. 8, Nr. 5, Mai 1972, NEW YORK, US, Seiten 1032-1035, XP000616926 R.V. BELINSKAYA, ET AL.: "Pyrolysis of lithium o-trifluoromethoxybenzenesulphonate and o-(trifluoromethylthio)benzenesulphonate" in der Anmeldung erwähnt

## Beschreibung

Die Erfindung betrifft ein neues Verfahren zur Herstellung von 2-Trifluormethoxybenzolsulfonamid, welches als Ausgangsstoff für herbizid wirksame Verbindungen bekannt ist.

Es ist bekannt, daß man 2-Trifluormethoxy-benzolsulfonamid erhält, wenn man 2-Trifluormethoxy-benzolsulfochlorid mit Ammoniak umsetzt (vgl. Zh. Org. Khim. 8 (1972), 1023-1026 (russ.) alias J. Org. Chem. USSR 8 (1972), 1032-1035 (engl.) - zitiert in Chem. Abstracts 77:61964; vgl. auch US 4732711).

Diese Umsetzung verläuft zwar glatt und liefert das 2-Trifluormethoxy-benzolsulfonamid in hohen Ausbeuten und in guter Qualität; das als Ausgangsstoff benötigte Trifluormethoxy-benzolsulfochlorid muß jedoch zuvor über eine sogenannte Meerwein-Reaktion aus 2-Trifluormethoxy-anilin hergestellt werden, deren Durchführung im industriellen Bereich relativ hohen Aufwand erfordert. Das benötigte Vorprodukt 2-Trifluormethoxy-anilin ist zudem nur in begrenztem Maße verfügbar.

Es wurde nun gefunden, daß man 2-Trifluormethoxy-benzolsulfonamid der Formel (I) 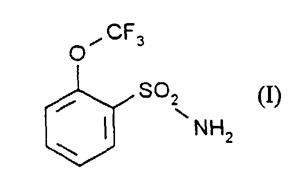 in sehr guten Ausbeuten und in hoher Reinheit erhält, wenn man halogenierte Trifluormethoxy-benzolsulfonamide der allgemeinen Formel (II), 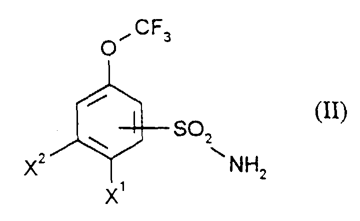 in welcher
- X¹: für Halogen steht und
- X²: für Wasserstoff oder Halogen steht,
mit Wasserstoff in Gegenwart eines Katalysators und in Gegenwart eines Verdünnungsmittels sowie gegebenenfalls in Gegenwart eines Säureakzeptors bei Temperaturen zwischen 0°C und 200°C umsetzt ("dehalogeniert"), die hierbei gebildete Verbindung der Formel (I) im Falle "X²=Halogen" in üblicher Weise isoliert und im Falle "X²=H" durch Behandeln mit einer protisch polaren organischen Flüssigkeit in ein reines, kristallines Produkt überführt und durch Absaugen der flüssigen Komponenten isoliert.

Die allgemeine Formel (II) steht für die Formeln (IIA) und (IIB) , 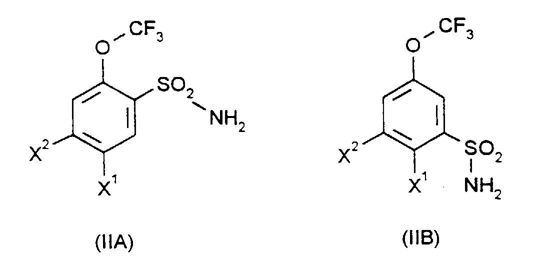 wobei
in Formel (IIA)
- X¹ und X²: die oben angegebenen Bedeutungen haben und
in Formel (IIB)
- X¹: die oben angegebene Bedeutung hat und X² nur für Wasserstoff steht.

Überraschenderweise wird die Trifluormethoxy-Gruppierung bei der hydrogenolytischen Dehalogenierung nicht verändert. Weiter ist es als sehr überraschend anzusehen, daß - im Falle "X²=H"- das unerwünschte, isomere Umsetzungsprodukt-3-Trifluormethoxy-benzolsulfonamid - durch einfaches Behandeln mit einer protisch polaren organischen Flüssigkeit in Lösung gebracht und somit leicht entfernt werden kann, während das erwünschte Produkt der Formel (I) dabei praktisch ungelöst bleibt und somit leicht durch Absaugen isoliert werden kann.

Das erfindungsgemäße Verfahren stellt somit eine wertvolle Bereicherung des Standes der Technik dar.

Die beim erfindungsgemäßen Verfahren zur Herstellung von 2-Trifluormethoxybenzolsulfonamid als Ausgangsstoffe zu verwendenden halogenierten Trifluormethoxy-benzolsulfonamide sind durch die Formel (II) allgemein definiert.

In der Formel (II) steht
- X¹: vorzugsweise für Chlor oder Brom, und
- X²: vorzugsweise für Wasserstoff oder Chlor.

Im Falle "X²=H" werden die Ausgangsstoffe als Gemisch der Verbindungen der Formeln (IIA) und (IIB) eingesetzt, wobei der Anteil der Verbindung der Formel (IIA) vorzugsweise größer als 60 % ist. Im Falle "X²=Halogen" entsprechen die Ausgangsstoffe der Formel (IIA).

Die Ausgangsstoffe der Formel (II, mit X²=H) sind bekannt und/oder können nach an sich bekannten Verfahren hergestellt werden (vgl. EP-A 23 422, EP-A 64 322). Die Ausgangsstoffe der Formel (II, mit X²=Halogen) sind neu und sind auch Gegenstand der Erfindung (vgl. Herstellungsbeispiele).

Das erfindungsgemäße Verfahren wird in Gegenwart eines Verdünnungsmittels durchgeführt. Als Verdünnungsmittel kommen vorzugsweise Wasser und organische Lösungsmittel in Betracht, insbesondere Alkohole, wie Methanol, Ethanol, n- oder i-Propanol, n-, i-, s- oder t-Butanol, Ether, wie Methyl-t-butylether, Methyl-t-pentylether, Ethylengylcol-dimethylether oder Tetrahydrofuran, Etheralkohole, wie Ethylenglycol-monomethylether oder -monoethylether, ferner Kohlenwasserstoffe, wie Hexan, Cyclohexan, Methylcyclohexan, Toluol oder Xylole, sowie auch Gemische der genannten Lösungsmittel.

Alkohole, insbesondere Methanol und Ethanol, werden als Verdünnungsmittel bei der Durchführung des erfindungsgemäßen Verfahrens ganz besonders bevorzugt eingesetzt.

Das erfindungsgemäße Verfahren wird in Gegenwart eines Katalysators durchgeführt. Als Katalysatoren kommen vorzugsweise die bei katalytischen Hydrierungen üblichen Metall-Katalysatoren - gegebenenfalls auf geeigneten Trägermaterialien - in Betracht. Hierzu gehören vorzugsweise (Raney-)Cobalt, (Raney-)Nickel, Palladium und Platin (letztere gegebenenfalls auf einem Trägermaterial wie z.B. Aktivkohle, Tonerde, Kieselgur oder Aluminiumoxid).

Palladium auf Aktivkohle wird als Katalysator beim erfindungsgemäßen Verfahren besonders bevorzugt eingesetzt.

Das erfindungsgemäße Verfahren wird gegebenenfalls in Gegenwart eines Säureakzeptors durchgeführt. Als Säureakzeptoren kommen im allgemeinen die üblichen anorganischen oder organischen Basen in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calciumhydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Trioctylamin, Tridodecylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo[5,4,0]-undec-7-en (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 20°C und 150°C, insbesondere zwischen 40°C und 120°C.

Das erfindungsgemäße Verfahren wird im allgemeinen unter Normaldruck oder bei erhöhtem Druck, vorzugsweise zwischen 1 bar und 100 bar, insbesondere zwischen 1 bar und 50 bar, durchgeführt.

In einer bevorzugten Ausführungsform des erfindungsgemäßen Verfahrens wird die Ausgangsverbindung der Formel (IIA, mit X²=Halogen) bzw. das Gemisch der Ausgangsverbindungen der Formeln (IIA, mit X²=H) und (IIB) in einem geeigneten Verdünnungsmittel vorgelegt, ein Katalysator und gegebenenfalls ein Säureakzeptor dazu gegeben und - vorzugsweise bei erhöhtem Druck und bei erhöhter Temperatur - auf übliche Weise hydriert.

Wenn die Hydrierung beendet ist, wird der restliche Wasserstoff mit Stickstoff verdrängt, die Mischung gegebenenfalls mit Wasser verdünnt und filtriert. Das Filtrat wird eingeengt und der Rückstand [welcher im Falle X²=H im wesentlichen aus einem Gemisch von 2-Trifluormethoxy-benzolsulfonamid und 3-Trifluormethoxy-benzolsulfonamid besteht] mit etwa der doppelten Gewichtsmenge einer protisch polaren organischen Flüssigkeit behandelt, d.h. verrührt.

Als protisch polare organische Flüssigkeiten kommen hierbei insbesondere gegebenenfalls substituierte Hydroxyalkylverbindungen in Betracht. Hierzu gehören vorzugsweise Alkohole, wie Methanol, Ethanol sowie jeweils geradkettige oder verzweigte Propanole, Butanole, Pentanole und Hexanole, ferner auch Alkoxyalkohole, wie Methoxyethanol und Ethoxyethanol.

n- und i-Propanol sowie n-, i-, s- und t-Butanol werden hierbei ganz besonders bevorzugt verwendet.

Das bei dieser Verfahrensweise kristallin anfallende 2-Trifluormethoxy-benzolsulfonamid der Formel (I) wird dann durch Absaugen isoliert.

Die nach dem erfindungsgemäßen Verfahren herzustellende Verbindung 2-Trifluormethoxy-benzolsulfonamid der Formel (I) kann als Zwischenprodukt zur Herstellung von herbizid wirksamen Verbindungen verwendet werden (vgl. US 4 732 711).

### Herstellungsbeispiele:

### Beispiel 1

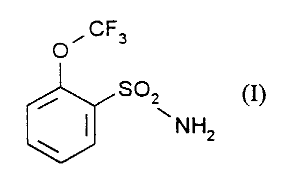

71,6 g eines Gemisches aus 2-Trifluormethoxy-5-chlor-benzolsulfonamid (Anteil: 75%) und 5-Trifluormethoxy-2-chlor-benzolsulfonamid (Anteil: 25%) - Gehalt an beiden Verbindungen zusammen: 71% - wird in 500 ml Ethanol aufgenommen, mit 5 g Palladium auf Aktivkohle (5%ig) versetzt und 20 Stunden bei einer Temperatur von ca. 100°C und einem Anfangsdruck von ca. 35 bar hydriert. Nach Durchleiten von Stickstoff und Abfiltrieren des Katalysators wird das Filtrat eingeengt, der Rückstand mit 260 ml n-Butanol verrührt und das kristallin angefallene Produkt durch Absaugen isoliert.

Man erhält 37,8 g (85% der Theorie) 2-Trifluormethoxy-benzolsulfonamid vom Schmelzpunkt 186°C.

### Beispiel 2

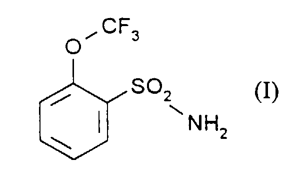

13,1 g eines Gemisches aus 2-Trifluormethoxy-5-brom-benzolsulfonamid (Anteil: 86%) und 5-Trifluormethoxy-2-brom-benzolsulfonamid (Anteil: 14%) - Gehalt an beiden Verbindungen zusammen: 76,1% - werden in 40 ml Methanol aufgenommen und mit einer Lösung von 2,3 g Kaliumhydroxid in 150 ml Methanol versetzt. Dann wird die Mischung mit 1 g Palladium auf Aktivkohle (5%ig) versetzt und 20 Stunden bei einer Temperatur von ca. 50°C und einem Anfangsdruck von ca. 35 bar hydriert Nach Durchleiten von Stickstoff und Abfiltrieren des Katalysators wird das Filtrat eingeengt, der Rückstand in Wasser aufgenommen, mit Essigsäureethylester geschüttelt, die organische Phase abgetrennt, mit Natriumsulfat getrocknet und filtriert. Das Filtrat wird eingeengt, der Rückstand mit 40 ml n-Butanol verrührt und das kristallin angefallene Produkt durch Absaugen isoliert.

Man erhält 6,7 g (89% der Theorie) 2-Trifluormethoxy-benzolsulfonamid vom Schmelzpunkt 186°C.

### Beispiel 3

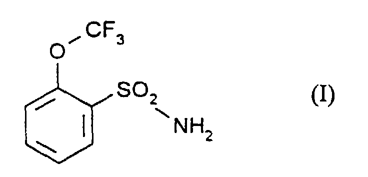

Zu einer Lösung von 15,5 g (0,05 mol) 2-Trifluormethoxy-4,5-dichlor-benzolsulfonamid in 60 ml Methanol werden eine Lösung von 5,6 g (0,1 mol) Kaliumhydroxid in 100 ml Methanol sowie 0,5 g Palladium auf Aktivkohle (5%ig) hinzugegeben. In einem Autoklaven wird 20 Stunden bei einer Temperatur von 75°C und einem Anfangsdruck von 35 bar hydriert. Zur Aufarbeitung wird der Katalysator abfiltriert, das Filtrat mit Wasser versetzt und neutral gestellt. Nach Abdestillieren des Methanols wird filtriert und getrocknet.

Man erhält 9,65 g (= 80 % der Theorie) 2-Trifluormethoxy-benzolsulfonamid von Schmelzpunkt 185°C.

### Herstellung der Ausgangsverbindung der Formel (IIA, mit X¹=X²=Cl)

### a) Stufe 1

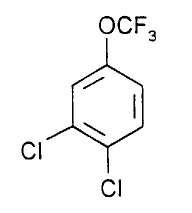

3.4-Dichlor-trifluormethoxybenzol

In einem VA-Autoklaven werden 200 g (1,22 mol) 3.4-Dichlorphenol und 800 ml Fluorwasserstoff bei 0°C vorgelegt und 800 ml Tetrachlormethan zugefügt. Nach Aufdrücken von 15 bar Stickstoff wird unter intensivem Rühren auf 116 bis 120°C erhitzt und der gebildete Chlorwasserstoff bei 28 bar entspannt. Die Chlorwasserstoffentwicklung ist nach 7 Stunden beendet, worauf der überschüssige Fluorwasserstoff zusammen mit gebildetem Trichlorfluormethan und überschüssigem Tetrachlormethan abdestilliert wird. Die Destillation des Produktgemisches bei 60 bis 86°C/17 mbar liefert 213 g Destillat, das zu 19,2 % auf 3.4-Dichlor-trifluormethoxybenzol und 80,1 % aus 3.4-Dichlor-difluorchlormethoxybenzol besteht.

Das Gemisch wird zusammen mit 100 ml Fluorwasserstoff und 1 ml Antimonpentachlorid für 3 Stunden auf 125°C erhitzt und der gebildete Chlorwasserstoff bei 25 bar entspannt. Die Destillation liefert 155 g (= 53 % der Theorie) 3.4-Dichlor-trifluormethoxybenzol mit einem Siedepunkt von 62°C bei 17 mbar; Gehalt nach GC: 99,8 %

### b) Stufe 2

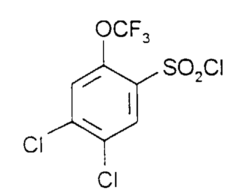

2-Trifluormethoxy-4.5-dichlor-benzolsulfochlorid

Zu 85 ml (1,28 mol) Chlorsulfonsäure werden innerhalb einer Stunde bei 0°C 50 g (0,216 mol) 3.4-Dichlor-trifluormethoxybenzol getropft. Nach einer Stunde bei 0°C werden 17 g Thionylchlorid zugetropft und anschließend wird auf 40°C erwärmt. Es tritt stoßweise Gasentwicklung auf.

Die Reaktionsmischung wird noch 20 Stunden nachgerührt. Dann wird im Vakuum das nicht umgesetzte Thionylchlorid abdestilliert und der auf 20°C gekühlte Rückstand auf 150 g Eis ausgetragen. Das Produkt wird in Dichlormethan aufgenommen und zweimal mit Wasser gewaschen. Die Lösung wird destilliert. Im Siedebereich von 96 bis 102°C bei 0,4 mbar gehen 57 g Produkt über, Gehalt nach GC: 99 %, das entspricht einer Ausbeute von 80 % der Theorie.

Diese Verbindung ist neu und ebenfalls Gegenstand der Erfindung.

### c) Stufe 3

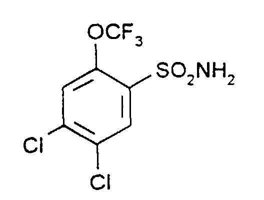

2-Trifluormethoxy-4.5-dichlor-benzolsulfonamid

Zu 200 ml einer 25 %igen Ammoniak-Lösung werden bei 20°C 57 g (0,173 mol) 2-Trifluormethoxy-4.5-dichlor-benzolsulfochlorid zudosiert. Nach 1-stündigem Nachrühren wird der Feststoff abgesaugt, anschließend mit Wasser nachgewaschen und getrocknet. Ausbeute 44 g (= 83 % der Theorie); Schmelzpunkt 181 bis 185°C.

Diese Verbindung ist gleichfalls neu und Gegenstand der Erfindung.

## Patentansprüche

1. Verfahren zur Herstellung von 2-Trifluormethoxy-benzolsulfonamid der Formel (I), 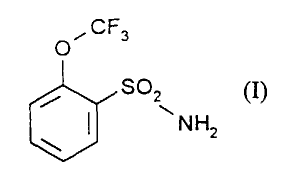 dadurch gekennzeichnet, daß man halogenierte Trifluormethoxy-benzolsulfonamide der allgemeinen Formel (II), 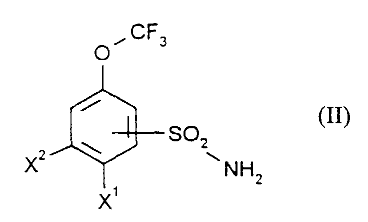 in welcher
X¹ für Halogen steht und
X² für Wasserstoff oder Halogen steht,
mit Wasserstoff in Gegenwart eines Katalysators und in Gegenwart eines Verdünnungsmittels sowie gegebenenfalls in Gegenwart eines Säureakzeptors bei Temperaturen zwischen 0°C und 200°C umsetzt (" dehalogeniert"), die hierbei gebildete Verbindung der Formel (I) im Falle "X²=Halogen" in üblicher Weise isoliert und im Falle "X²=H" durch Behandeln mit einer protisch polaren organischen Flüssigkeit in ein reines, kristallines Produkt überführt und durch Absaugen der flüssigen Komponenten isoliert.

2. Verfahren nach Anspruch 1, dadurch gekennzeichnet, daß man die Umsetzung bei Temperaturen zwischen 20°C und 150°C durchführt.

3. Verfahren nach Anspruch 1, dadurch gekennzeichnet, daß man die Umsetzung bei Temperaturen zwischen 40°C und 120°C durchführt.

4. Verfahren nach Anspruch 1, dadurch gekennzeichnet, daß man die Umsetzung bei einem Druck zwischen 1 bar und 100 bar, insbesondere zwischen 1 bar und 50 bar durchführt.

5. Verfahren nach Anspruch 1, dadurch gekennzeichnet, daß man als Katalysator Palladium auf Aktivkohle verwendet.

6. Verfahren nach Anspruch 1, dadurch gekennzeichnet, daß man als Verdünnungsmittel einen Alkohol, insbesondere Methanol oder Ethanol verwendet.

7. Verfahren nach Anspruch 1, dadurch gekennzeichnet, daß man als protisch polare Flüssigkeit eine gegebenenfalls substituierte Hydroxyalkylverbindung, vorzugsweise einen Alkohol oder Alkoxyalkohol, verwendet.

8. Verfahren nach Anspruch 7, dadurch gekennzeichnet, daß man als Alkohol n- oder i-Propanol oder n-, i-, s- oder t-Butanol verwendet.

9. Die Verbindungen 2-Trifluormethoxy-4,5-dichlor-benzolsulfochlorid und 2-Trifluormethoxy-4,5-dichlor-benzolsulfonamid.

## Claims

1. Process for the preparation of 2-trifluoromethoxy-benzenesulphonamide, of the formula (I), 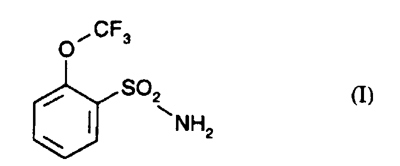 characterized in that halogenated trifluoromethoxy-benzenesulphonamides of the general formula (II) 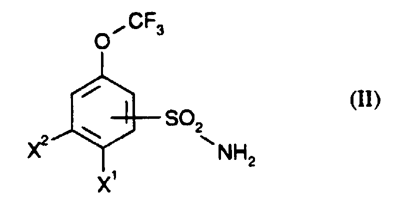 in which
X¹ represents halogen and
X² represents hydrogen or halogen
are reacted with hydrogen in the presence of a catalyst and in the presence of a diluent and, if appropriate, in the presence of an acid acceptor at temperatures between 0°C and 200°C ("dehalogenated"), the resulting compound of the formula (I) is isolated in the customary manner in the event that "X²=halogen" and converted into a pure, crystalline product by treating it with a protic polar organic liquid and isolated by removing the liquid component by means of filtration with suction in the event that "X²=H".

2. Process according to Claim 1, characterized in that the reaction is carried out at temperatures between 20°C and 150°C.

3. Process according to Claim 1, characterized in that the reaction is carried out at temperatures between 40°C and 120°C.

4. Process according to Claim 1, characterized in that the reaction is carried out at a pressure between 1 bar and 100 bar, in particular between 1 bar and 50 bar.

5. Process according to Claim 1, characterized in that palladium on charcoal is used as the catalyst.

6. Process according to Claim 1, characterized in that an alcohol, in particular methanol or ethanol, is used as the diluent.

7. Process according to Claim 1, characterized in that an optionally substituted hydroxyalkyl compound, preferably an alcohol or alkoxyalcohol, is used as the protic polar liquid.

8. Process according to Claim 7, characterized in that n- or i-propanol or n-, i-, s- or t-butanol is used as the alcohol.

9. The compounds 2-trifluoromethoxy-4,5-dichloro-benzene sulphochloride and 2-trifluoromethoxy-4,5-dichloro-benzenesulphonamide.

## Revendications

1. Procédé de production de 2-trifluorométhoxy-benzènesulfonamide de formule (I) 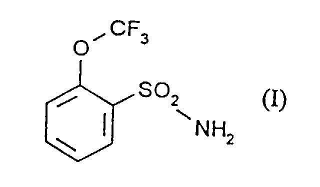 caractérisé en ce qu'on fait réagir ("déshalogéner") des trifluorométhoxybenzènesulfonamides halogénés de formule générale (II) 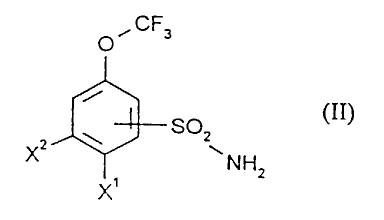 dans laquelle
X¹ est un halogène et
X² est l'hydrogène ou un halogène,
avec l'hydrogène en présence d'un catalyseur et en présence d'un diluant ainsi que, le cas échéant, en présence d'un accepteur d'acide, à des températures comprises entre 0°C et 200°C, on isole de façon classique le composé ainsi formé, de formule (I) dans le cas "X²=halogène" et, dans le cas "X²-H", on le transforme par traitement avec un liquide organique à polarité protique, en un produit cristallin pur et qu'on isole par succion des composants liquides.

2. Procédé suivant la revendication 1, caractérisé en ce qu'on conduit la réaction à des températures comprises entre 20°C et 150°C.

3. Procédé suivant la revendication 1, caractérisé en ce qu'on conduit la réaction à des températures comprises entre 40°C et 120°C.

4. Procédé suivant la revendication 1, caractérisé en ce qu'on conduit la réaction à une pression comprise entre 1 bar et 100 bars, notamment entre 1 bar et 50 bars.

5. Procédé suivant la revendication 1, caractérisé en ce qu'on utilise comme catalyseur du palladium sur du charbon actif.

6. Procédé suivant la revendication 1, caractérisé en ce qu'on utilise comme diluant un alcool, en particulier le méthanol ou l'éthanol.

7. Procédé suivant la revendication 1, caractérisé en ce qu'on utilise comme liquide à polarité protique un composé hydroxyalkylé éventuellement substitué, de préférence un alcool ou un alkoxy-alcool.

8. Procédé suivant la revendication 7, caractérisé en ce qu'on utilise comme alcool le n-propanol, l'isopropanol, le n-butanol, l'isobutanol, le sec.-butanol ou le tertiobutanol.

9. Le sulfochlorure de 2-trifluorométhoxy-4,5-dichlorobenzéne et le 2-trifluorométhoxy-4,5-dichlorobenzènesulfonamide.
